# EUROPEAN PATENT SPECIFICATION

(11) **EP 2 659 800 B1**
(45) Date of publication and mention of the grant of the patent: **28.02.2018**
(21) Application number: 13000248.8
(22) Date of filing: 17.01.2013
(51) Int. Cl.: A45C 5/02, D05B 93/00

(54) **Reinforced textile**
Verstärkter Stoff
Textile renforcé

(30) Priority: 18.01.2012 AU 2012900188
(43) Date of publication of application: 06.11.2013
(73) Proprietor: MRM HK LIMITED, Hong Kong (CN)
(72) Inventor: Schlipper, Robert Wesley, Hong Kong (CN)
(74) Representative: Oxley, Robin John George

(56) References cited:
- EP-A1- 0 878 143
- WO-A1-03/077692
- FR-A- 1 601 035

## Description

### Technical field

The present invention relates to a reinforced textile, which is particularly suitable for the construction of soft security baggage.

### Background of the invention

EP0878143 relates to a security device for luggage according to the abstract of which the security device, for a backpack, comprises a cover in the form of a wire netting that is held snugly to the backpack by a drawline. The drawline is taughtened and locked tight with a locking mechanism. The cover prevents tampering with or removing the backpack from the wearer, and especially the slicing open of the backpack to remove its contents.

The use of reinforcement such as wire mesh in the manufacture of flexible or soft security baggage provides an increased level of security against theft, particularly that which achieved by slicing open the bag. The mesh can be held adjacent the fabric or leather walls of the baggage for greatly enhanced security, while still retaining the advantages of traditional un-reinforced products. For bags of this construction, such as backpacks, it is convenient to have a fabric outer forming the main body and providing a waterproof shell about the mesh layer. To prevent the bag's contents snagging, or being damaged by the mesh exposed on the inside, a fabric liner is used and is typically of relatively lighter material than the outer.

The mesh may be formed in the shape of the bag from a single wire strand, or formed as panels. Fastenings may join adjacent portions of the strand, with the strand extending generally longitudinally and being turned back upon itself to form closed loops at each of the opposing edges of the mesh. Due to the construction of the mesh bag or panel in this manner, when the mesh is in a relaxed state the mesh tends to collapse, closing the open spaces between the strands. In the past, the baggage, or separate panels from which the bag is assembled, were a composite reinforced textile, comprising inner and outer bags laminated together to enclose a mesh bag, or two panels laminated together with the mesh panel held therebetween.

Bonding the layers in this manner avoids the problems resulting from the mesh separating from the outer and the liner due to the tendency of the mesh to collapse. However, the manufacture of laminated baggage or panels is relatively costly. It is an object of the present invention to overcome or substantially ameliorate the above disadvantages or more generally to provide an improved reinforced textile for the construction of soft baggage.

### Disclosure of the Invention

According to one aspect not part of the present invention there is provided a reinforced textile, comprising:
a sheet of fabric;
a reinforcing strand arrayed in a pattern overlying a surface of the fabric, and stitching extending lengthwise along the reinforcing strand and fixing the reinforcing strand to the fabric.

Preferably the reinforcing strand is substantially continuous and comprises a metal strand. Optionally the reinforcing strand may comprise multiple strands, such as a wire, or other material including relatively high-strength strands, such as carbon fibres. As required, on a single sheet of fabric different lengths of the continuous reinforcing strand may be stitched to opposing sides, or separate reinforcing strands may be stitched to opposing sides of the fabric. A cover sheet may cover the reinforcing strand, and optionally, the cover sheet may be bonded to the sheet of fabric. The cover sheet may be a fabric sheet.

Preferably the pattern comprises a net- or mesh-like pattern. Preferably the reinforcing strand overlies itself it at intersections or nodes in the mesh-like pattern.

Preferably the mesh-like pattern is non-woven, and the reinforcing strand is not looped, knotted, crimped or otherwise connected to itself at the intersections.

Preferably the stitching comprises a lockstitch, wherein one thread of the lockstitch crosses back and forth over the reinforcing strand, and the other thread of the lockstitch extends longitudinally in the direction of the reinforcing strand.

Preferably the fabric is woven, but it may alternatively be knitted. Preferably the reinforced textile comprises a panel for the construction of baggage, particularly a lining panel for the inside of baggage. Another aspect not part of the invention comprises security baggage incorporating one or more panels comprising a reinforced composite textile as described above. Another aspect not part of the invention comprises a method of manufacturing a reinforced composite textile, comprising: sewing longitudinally along a continuous reinforcing strand to secure the reinforcing strand to a fabric in a pattern overlying a surface of the fabric.

Preferably the sewing is performed by an embroidering machine which simultaneously feeds the reinforcing strand together with first and second threads, the first and second threads forming a lockstitch, the first thread crossing back and forth over the reinforcing strand, and the second thread extending longitudinally in the direction of the reinforcing strand. Alternatively, other stitches may be employed to secure the reinforcing strand and fabric. Another aspect not part of the invention provides a method of reinforcing a fabric panel, comprising: fastening an elongate reinforcement to the panel, the reinforcement following a boustrophedonic path comprising a plurality of sets of alternating first and second reinforcement lengths, each reinforcement length having a wave shape, each first and second reinforcement length having a common central axis, and wherein crests and troughs of adjacent sets overlap one another. Another aspect not part of the invention provides a method of reinforcing a fabric panel, comprising: fastening an elongate reinforcement to the panel, the reinforcement following a boustrophedonic path comprising a plurality of alternating reinforcement lengths, each reinforcement length having a wave shape and a respective central axis, and wherein crests and troughs of adjacent reinforcement lengths overlap one another. The present invention is a reinforced textile according to claim 1. Preferably the wave shape is substantially sinusoidal. Preferably the amplitude and frequency of the wave shape are substantially constant for a plurality of the reinforcement lengths.

Preferably the reinforcing strand is continuous and is continuously or intermittently fastened to the fabric by one of: stitching, adhesive and welding.

Preferably the stitching comprises a lock stitch continuous with the reinforcing strand. Preferably one thread of the lockstitch crosses back and forth over the reinforcing strand, and the other thread of the lockstitch extends longitudinally in the direction of the reinforcing strand.

Optionally, the reinforcing strand may be enclosed in a tape, and the lock stitch connects the tape to the fabric.

Optionally the adhesive comprises a longitudinally continuous or intermittent adhesive bead bonding the reinforcing strand to the fabric. Optionally the adhesive further comprises adhesive tape continuous with the reinforcing strand or a plurality of lengths of adhesive tape, wherein the reinforcing strand is bonded between the fabric and the adhesive tape.

Optionally the reinforcing strand is coated in a fusible material and the welding comprises longitudinally continuous or intermittent ultrasonic welding of the fusible material to the fabric.

Preferably the reinforcing strand comprises a metal, or another cut-resistant material.

Preferably the reinforced textile comprises a panel for the construction of baggage, particularly a lining panel for the inside of baggage

In still another aspect the invention provides security baggage incorporating one or more panels comprising a reinforced composite textile according to claim 1. Preferably the one or more panels are assembled to form a liner reinforced by the reinforcing strands, the liner being received in a complementary shell to form a two-layer construction in which the reinforcing strands are disposed between the fabric of the liner and that of the shell.

Preferably the mesh pattern of the reinforcing strands terminates adjacent seams or fold lines in the liner.

This invention provides a reinforced textile which is effective which can be incorporated into the construction of security baggage, at a reduced manufacturing cost but without compromising performance.

### Brief Description of the Drawings

Preferred forms of the present invention will now be described by way of example with reference to the accompanying drawings, wherein:
Figure 1 is a schematic section through the reinforced textile of the invention;
Figure 2 is a schematic plan view of the reinforced textile of Fig. 1, showing a first preferred pattern of the reinforcing strand;
Figure 3 is a schematic plan view of the reinforced textile of Fig. 1, showing a second preferred pattern of the reinforcing strand;
Figure 4 illustrates a liner panel assembly with panels reinforced according to the invention, and
Figure 5 illustrates a bag liner assembled from the liner panel assembly of fig. 4.

### Description of the Preferred Embodiments

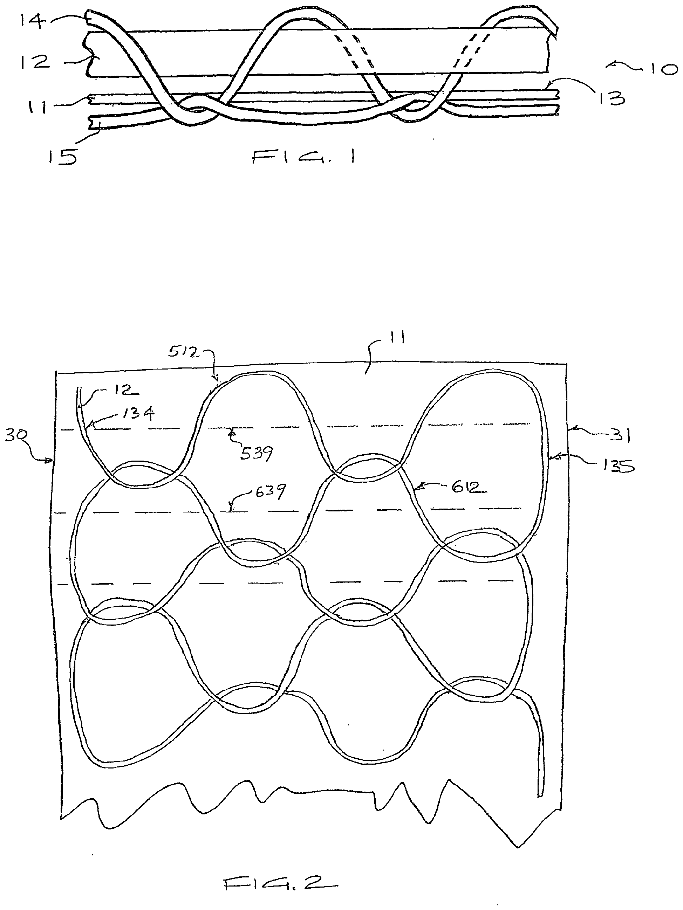

Referring to Fig. 1, a reinforced textile 10 may be made from a sheet of fabric 11 to which a continuous reinforcing strand 12 is sewn. The reinforcing strand 12 overlies a surface 13 of the fabric 11 and is fixed to the fabric 11 by a lockstitch formed of an upper thread 14 and lower thread 15. The lockstitch may be a zigzag stitch in which the upper thread 14 crosses back and forth over the reinforcing strand 12, and the lower thread extends longitudinally in the direction of the reinforcing strand 12. The reinforcing strand 12 preferably comprises a multi-stranded metal wire, and the threads 14, 15 may comprise multi-filament threads. It will be appreciated that the illustration is schematic, and the scale and the space shown between the surface 13 and reinforcing strand 12, are not representative of the actual geometry.

As shown in Fig. 2, the reinforcing strand 12 may be one continuous length arrayed in a regular, mesh-like pattern and held by the threads 14, 15 (which are not shown in Fig. 2). The mesh-like pattern of Fig. 2 is of a first preferred type and may be formed with the reinforcing strand 12 extending in a wave-like form of constant frequency and amplitude, back and forth generally transversely in direction 21 between opposing edges 30, 31, the reinforcing strand 12 being turned back upon itself to form closed loops at each of the opposing edges.

The reinforcing strand 12 is continuously fed, as from a reel (not shown), to an embroidering machine head (not shown) by which the reinforcing strand 12 is stitched to the panel. The embroidering machine head traces the mesh pattern while stitching and placing the reinforcing strand 12 at the same rate. The reinforcing strand 12 generally follows a boustrophedonic path extending back and forth between the edges 30, 31 and comprises reinforcement lengths that alternate in direction. The first reinforcement length 512 extends left to right (with reference to Fig. 2), starting for instance at point 134 and extending to point 535 where it meets the second reinforcement length 612 that extends right to left, underlying the length 512 at nodes or intersections. The reinforcement lengths 512, 612 may have a substantially sinusoidal shape of the same amplitude and frequency. Reinforcement lengths 512 has a central axis 539 to either side of which the reinforcement length 512 extends, while reinforcement lengths 612 has a central axis 639 to either side of which the reinforcement length 512 extends. As Fig. 2 illustrates, the crests 40 and the troughs 41 of wave-shaped adjacent reinforcement lengths 512, 612 overlap one another, specifically the troughs 41 of length 512 overlap the crests 40 of the length 612. By overlapping the reinforcing strand 12 in the manner of Fig. 2, there is no path for a blade to pass between and separate the crests, unless the plane of the blade lies generally parallel to that of the textile 10, so the bag reinforced with this textile 10 is secured against being slashed open by a blade, even for a cut along a join in the mesh.

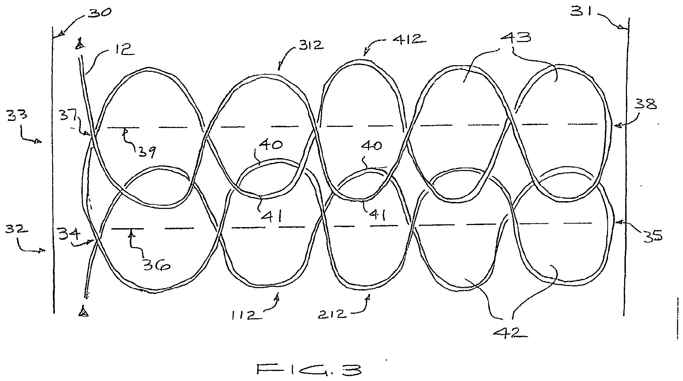

Fig. 3 illustrates a second preferred method of reinforcing a fabric panel with a mesh pattern that extends between opposing edges 30, 31. The reinforcing strand 12 is continuously fed, as from a reel (not shown), to an embroidering machine head (not shown) by which the reinforcing strand 12 is stitched to the panel. The embroidering machine head traces the mesh pattern while stitching and placing the reinforcing strand 12 at the same rate. The reinforcing strand 12 generally follows a boustrophedonic path extending back and forth between the edges 30, 31 and comprises first and second reinforcement lengths that alternate in direction and are arranged in a plurality of sets, two sets of which, sets 32 and 33, are shown in Fig. 3. Set 32 comprises reinforcement lengths 112, 212, while set 33 comprises like reinforcement lengths 312, 412 of the continuous reinforcing strand 12.

The first reinforcement length 112 of set 32 extends left to right (with reference to Fig. 3), starting for instance at point 34 and extending to point 35 where it meets the second reinforcement length 212 of set 32 that extends right to left, overlying the length 112 at nodes or intersections. The reinforcement lengths 112, 212 may have a substantially sinusoidal shape of the same amplitude and frequency, and a common central axis 36 to either side of which the reinforcement lengths 112, 212 extend. In this manner the resulting set 32 appears as a row of contiguous mesh openings or loops 42.

The set 33 is formed in a like manner from reinforcement lengths 312, 412 and appears as a row of contiguous loops 43. The first reinforcement length 312 of set 33 extends left to right, starting at point 37 and extending to point 38 where it meets the second reinforcement length 412 of set 33 that extends right to left, overlying the length 312 at nodes or intersections. The reinforcement lengths 312, 412 may have a like substantially sinusoidal shape extending either side of common central axis 39 at the same amplitude and frequency. As Fig. 3 illustrates, the loops 42, 43 of the sets 32, 33 overlap one another, more specifically the crests 40 of set 32 overlap the troughs 41 of set 33, the troughs 41 overlying the previously formed crests 40.
It will be understood that the mesh pattern of Fig. 3 can be varied to cover panels of varying shapes, not only those which have parallel edges 30, 31 or which are rectangular. In particular, the shape of the loops 42, 43 adjacent the edges of the panel can be varied, so as to allow the reinforcing strand 12 to extend adjacent to the edges.

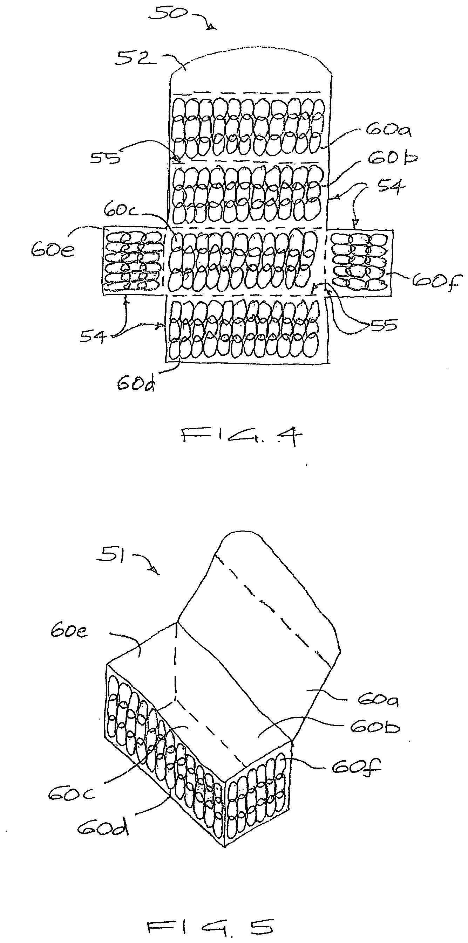

Figs. 4 and 5 illustrate an exemplary construction of a reinforced liner for a bag, showing the panel assembly 50 and assembled liner 51 respectively. The panel assembly 50 may comprise six rectangular panels 60a-60f, each reinforced according to the invention with a mesh pattern, and formed on one side of a single sheet 52 of fabric. The mesh pattern may extend adjacent to, or contiguous with, the seams 54 and fold lines 55 (shown as broken lines) of each panel 60a-60f. As the reinforcing strand 12 stiffens the panels, terminating the mesh pattern adjacent to the seams and fold lines allows "sharper" corners to be formed along these edges than if the reinforcing strands themselves spanned across the seams or fold lines and thus had to be bent around the corners. The liner 51 has the reinforcement mesh pattern on its outer side and by inserting the liner 51 into a complementary outer bag shell (not shown) a two-layer bag may be formed, in which the reinforcing strands 12 are sandwiched between panels of the liner 51 and shell, providing a lightweight, simple, water-proof reinforced construction.
Aspects of the present invention have been described by way of example only and it should be appreciated that modifications and additions may be made thereto without departing from the scope of the appended claims.

## Claims

1. A reinforced textile (10), comprising:
a sheet (52) of fabric (11);
a continuous strand (12) arrayed in a pattern overlying a surface (13) of the fabric (11) and fastened to the fabric (11) continuously or intermittently, the continuous strand (12) following a boustrophedonic path and comprising a plurality of sets of alternating first and second reinforcement lengths (112, 212, 312, 412, 512, 612), each reinforcement length having a wave shape with crests (40) and troughs (41), **characterised in that**
a) each reinforcement length has a respective central axis (539, 639), wherein crests (40) and troughs (41) of adjacent reinforcement lengths (512, 612) overlap one another, or
b) the first and second reinforcement length of each set have a common central axis (36, 39), and wherein crests and troughs of adjacent sets overlap one another.

2. The reinforced textile (10) of claim 1 wherein the wave shape is sinusoidal.

3. The reinforced textile (10) of claim 2 wherein the amplitude and frequency of the wave shape are constant for a plurality of the reinforcement lengths (112, 212, 312, 412, 512, 612).

4. The reinforced textile (10) of any one of claims 1 to 3 wherein the continuous strand (12) is fastened to the fabric (11) by one of: stitching, adhesive and welding.

5. The reinforced textile (10) of any one of claims 1 to 3 wherein the continuous strand (12) is continuously fastened to the fabric (11) by a lock stitch continuous with the continuous strand (12).

6. The reinforced textile (10) of claim 5 wherein one thread of the lockstitch crosses back and forth over the continuous strand (12), and the other thread of the lockstitch extends longitudinally in the direction of the continuous strand (12).

7. The reinforced textile (10) of claim 4 wherein the adhesive comprises a longitudinally continuous or intermittent adhesive bead bonding the continuous strand (12) to the fabric (11).

8. The reinforced textile (10) of claim 4 wherein the adhesive further comprises adhesive tape continuous with the continuous strand (12) or a plurality of lengths of adhesive tape, wherein the continuous strand (12) is bonded between the fabric (11) and the adhesive tape.

9. The reinforced textile (10) of claim 4 wherein the continuous strand (12) is coated in a fusible material and the welding comprises longitudinally continuous or intermittent ultrasonic welding of the fusible material to the fabric (11).

10. The reinforced textile (10) of any one of claims 1 to 9 wherein the continuous strand (12) comprises a metal, or another cut-resistant material.

11. The reinforced textile (10) of any one of claims 1 to 10 wherein the reinforced textile (10) comprises a panel (60a-60f) for the construction of baggage, particularly a lining panel (60a-60f) for the inside of baggage.

12. Security baggage incorporating one or more panels (60a-60f) comprising a reinforced composite textile (10) as claimed in any one of claims 1 to 11.

13. Security baggage of claim 12 wherein the one or more panels (60a-60f) are assembled to form a liner reinforced by the continuous strands, the liner being received in a complementary shell to form a two-layer construction in which the continuous strands are disposed between the fabric (11) of the liner and that of the shell.

## Patentansprüche

1. Verstärkter Stoff (10), Folgendes beinhaltend:
ein Tuch (52) aus Gewebe (11);
einen durchgehenden Strang (12), angeordnet in einem Muster, welches eine Oberfläche (13) des Gewebes (11) überlagert und am Gewebe (11) durchgehend oder intermittierend befestigt ist, wobei der durchgehende Strang (12) einen ochsenwendigen Weg beschreibt und eine Vielzahl von Sätzen von alternierenden ersten und zweiten Verstärkungslängen (112, 212, 312, 412, 512, 612) besitzt, wobei jede Verstärkungslänge eine Wellenform mit Kämmen (40) und Tälern (41) besitzt, **dadurch gekennzeichnet, dass**
a) jede Verstärkungslänge eine jeweilige Mittelachse (539, 639) besitzt, worin Kämme (40) und Wellen (41) benachbarter Verstärkungslängen (512, 612) einander überlappen, oder
b) die erste und zweite Verstärkungslänge eines jeden Satzes eine gemeinsame Mittelachse (36, 39) besitzen, und wobei Kämme und Täler benachbarter Sätze einander überlappen.

2. Verstärkter Stoff (10) nach Anspruch 1, bei welchem die Wellenform eine Sinusform besitzt.

3. Verstärkter Stoff (10) nach Anspruch 2, bei welchem die Amplitude und Frequenz der Wellenform für eine Vielzahl von Verstärkungslängen (112, 212, 312, 412, 512, 612) konstant sind.

4. Verstärkter Stoff (10) nach einem der Ansprüche 1 bis 3, bei welchem der durchgehende Strang (12) am Gewebe (11) mit einem von Nähen, Kleben und Schweißen befestigt ist.

5. Verstärkter Stoff (10) nach einem der Ansprüche 1 bis 3, bei welchem der durchgehende Strang (12) durchgehend am Gewebe (11) mit einem Steppstich befestigt ist, der durchgehend mit dem durchgehenden Strang (12) ist.

6. Verstärkter Stoff (10) nach Anspruch 5, bei welchem ein Faden des Steppstichs vorwärts und rückwärts über den durchgehenden Strang (12) kreuzt und der andere Faden des Steppstichs sich längs in die Richtung des kontinuierlichen Strangs (12) erstreckt.

7. Verstärkter Stoff (10) nach Anspruch 4, bei welchem der Kleber eine in Längsrichtung durchgehende oder intermittierende Wulst beinhaltet, welche den durchgehenden Strang (12) am Gewebe (11) verklebt.

8. Verstärkter Stoff (10) nach Anspruch 4, bei welchem der Kleber zudem Folgendes beinhaltet: Klebeband, durchgehend mit dem durchgehenden Strang (12), oder eine Vielzahl von Längen von Klebeband, wobei der durchgehende Strang (12) zwischen dem Gewebe (11) und dem Klebeband verklebt wird.

9. Verstärkter Stoff (10) nach Anspruch 4, bei welchem der durchgehende Strang (12) mit einem schmelzbaren Material beschichtet ist und das Schweißen durchgehendes oder intermittierendes Ultraschall-Schweißen des schmelzbaren Materials an das Gewebe (11) beinhaltet.

10. Verstärkter Stoff (10) nach einem der Ansprüche 1 bis 9, bei welchem der durchgehende Strang (12) ein Metall oder ein anderes schnittfestes Material beinhaltet.

11. Verstärkter Stoff (10) nach einem der Ansprüche 1 bis 10, bei welchem der verstärkte Stoff (10) eine Platte (60a-60f) zur Konstruktion von Gepäckstücken beinhaltet, insbesondere einer Verkleidungsplatte (60a-60f) für das Innere von Gepäckstücken.

12. Sicherheitsgepäckstück, enthaltend eine oder mehrere Platten (60a-60f), beinhaltend einen verstärkten Komposit-Stoff (10) nach einem der Ansprüche 1 bis 11.

13. Sicherheitsgepäckstück nach Anspruch 12, bei welchem die eine oder die mehreren Platte(n) (60a-60f) zusammengebaut ist/sind, um eine Verkleidung, verstärkt durch die durchgehenden Stränge, zu bilden, wobei die Verkleidung in einer zusätzlichen Schale untergebracht ist, um eine zweischichtige Konstruktion zu bilden, in welcher die durchgehenden Stränge zwischen dem Gewebe (11) der Verkleidung und demjenigen der Schale angeordnet sind.

## Revendications

1. Textile renforcé (10), comprenant :
une bande (52) de tissu (11) ;
un brin continu (12) qui est agencé selon un motif qui recouvre une surface (13) du tissu (11) et qui est fixé au tissu (11) en continu ou de façon intermittente, le brin continu (12) suivant un parcours boustrophédonique et comprenant une pluralité de jeux de premières et secondes longueurs de renforcement alternées (112, 212, 312, 412, 512, 612), chaque longueur de renforcement présentant une forme ondulée avec des crêtes (40) et des creux (41), **caractérisé en ce que** :
a) chaque longueur de renforcement comporte un axe central respectif (539, 639), dans lequel des crêtes (40) et des creux (41) de longueurs de renforcement adjacentes (512, 612) se chevauchent les uns les autres, ou
b) les première et seconde longueurs de renforcement de chaque jeu comportent un axe central commun (36, 39), et dans lequel des crêtes et des creux de jeux adjacents se chevauchent les uns les autres.

2. Textile renforcé (10) selon la revendication 1, dans lequel la forme ondulée est sinusoïdale.

3. Textile renforcé (10) selon la revendication 2, dans lequel l'amplitude et la fréquence de la forme ondulée sont constantes pour une pluralité des longueurs de renforcement (112, 212, 312, 412, 512, 612).

4. Textile renforcé (10) selon l'une quelconque des revendications 1 à 3, dans lequel le brin continu (12) est fixé au tissu (11) par l'un parmi une piqûre, un adhésif et une soudure.

5. Textile renforcé (10) selon l'une quelconque des revendications 1 à 3, dans lequel le brin continu (12) est fixé en continu au tissu (11) au moyen d'un point noué qui est continu avec le brin continu (12).

6. Textile renforcé (10) selon la revendication 5, dans lequel un fil du point noué est croisé en va-et-vient au-dessus du brin continu (12), et l'autre fil du point noué s'étend longitudinalement dans la direction du brin continu (12).

7. Textile renforcé (10) selon la revendication 4, dans lequel l'adhésif comprend un cordon adhésif continu ou intermittent longitudinalement qui colle le brin continu (12) au tissu (11).

8. Textile renforcé (10) selon la revendication 4, dans lequel l'adhésif comprend en outre une bande adhésive qui est continue avec le brin continu (12) ou une pluralité de longueurs de bande adhésive, dans lequel le brin continu (12) est collé entre le tissu (11) et la bande adhésive.

9. Textile renforcé (10) selon la revendication 4, dans lequel le brin continu (12) est enduit d'un matériau fusible et le soudage comprend le soudage par ultrasons continu ou intermittent longitudinalement du matériau fusible sur le tissu (11).

10. Textile renforcé (10) selon l'une quelconque des revendications 1 à 9, dans lequel le brin continu (12) comprend un métal ou un autre matériau résistant aux coupures.

11. Textile renforcé (10) selon l'une quelconque des revendications 1 à 10, dans lequel le textile renforcé (10) comprend un panneau (60a-60f) pour la fabrication de bagages, en particulier un panneau de doublage (60a-60f) pour l'intérieur de bagages.

12. Bagage de sécurité incorporant un ou plusieurs panneau(x) (60a-60f) comprenant un textile composite renforcé (10) comme revendiqué selon l'une quelconque des revendications 1 à 11.

13. Bagage de sécurité selon la revendication 12, dans lequel les un ou plusieurs panneau(x) (60a-60f) est/sont assemblé(s) de manière à former une doublure renforcée par les brins continus, la doublure étant reçue dans une enveloppe complémentaire de manière à former une construction bicouche dans laquelle les brins continus sont disposés entre le tissu (11) de la doublure et celui de l'enveloppe.
